(19) Europäisches Patentamt
European Patent Office
Office européen des brevets

(11) Publication number: **0 149 475 B1**

(12) **EUROPEAN PATENT SPECIFICATION**

(45) Date of publication of patent specification: **18.04.90**

(51) Int. Cl.[5]: **A 61 K 31/44**

(21) Application number: **85100222.0**

(22) Date of filing: **11.01.85**

(54) **Medicaments for the treatment and prevention of liver damage.**

(30) Priority: **13.01.84 US 570712**

(43) Date of publication of application:
**24.07.85 Bulletin 85/30**

(45) Publication of the grant of the patent:
**18.04.90 Bulletin 90/16**

(84) Designated Contracting States:
**AT BE CH DE FR GB IT LI LU NL SE**

(56) References cited:
**EP-A-0 145 434**
**EP-B-0 087 156**
**DE-A-2 407 115**
**FR-A-2 248 028**
**CHEMICAL ABSTRACTS, vol. 90, no. 15, 9th April 1979, page 43, no. 115049y, Columbus, Ohio, US; N.SAKAMOTO et al.: "Inhibition of cyclic AMP phosphodiesterase by 2,6-dimethyl-4-(3-nitrophenyl)-1,4-dihydropyridine-3,5-dicarboxylic acid 3-2-(N-benzyl-N-methylamino)r ethyl ester 5-methyl ester hydrochloride (YC-93), a potent vasodilator" & BIOCHEM. PHARMACOL. 1978, 27(8), 1269-74**

**The file contains technical information submitted after the application was filed and not included in this specification**

(73) Proprietor: **YAMANOUCHI PHARMACEUTICAL CO. LTD.**
**No. 3-11 Nihonbashi-Honcho, 2-chome Chuo-ku**
**Tokyo (JP)**

(72) Inventor: **Garay, Gabriel L.**
**725 K. Blair Court**
**Sunnyvale California 94087 (US)**

(74) Representative: **Wuesthoff, Franz, Dr.-Ing. et al**
**Wuesthoff & Wuesthoff Patent- und Rechtsanwälte Schweigerstrasse 2**
**D-8000 München 90 (DE)**

(56) References cited:
**CHEMICAL ABSTRACTS, vol. 102, no. 13, 1st April 1985, page 71, no. 106248b, Columbus, Ohio, US; I.BUIKIS et al.: "Changes in the activity of hydrolytic enzymes in the liver of white rats under the effect of membrane-active substances" & MORFOL. OTSENKA REZIST. CHUVSTVITEL'NOSTI OPUKHOLEI KHIMIOTER, 1983, 102-23**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 99, no. 25, 19th December 1983, page 202, no. 207523n, Columbus, Ohio, US; A.MAJORE et al.: "Ability of some derivatives of 1,4-dihydropyridine to prevent allyl alcohol-induced toxicities in rat liver" & EKSP. MED.(RIGA) 1982, 14, 97-103

CHEMICAL ABSTRACTS, vol. 76, no. 19, 8th May 1972, no. 107917a, Columbus, Ohio, US; K.KUMSARS et al.: "Action of various 1,4-dihydropyridine and 1,6-dihydropyrimidine derivatives on the energy metabolism of normal and tumor cells" & BIOKHIMIYA 1971, 36(6), 1204-9

CHEMICAL ABSTRACTS, vol. 101, no. 17, 22nd October 1984, no. 143922s, Columbus, Ohio, US; I.OHATA et al.: "Low density lipoprotein-lowering and high density lipoprotein-elevating effects of nicardipine in rats" & BIOCHEM. PHARMACOL. 1984, 33(14), 2199-205

JR Mitchell et al. Seminars in liver disease I(2), 1981,143-150

H. Liehr et al.: Virchows Archiv B, Cell Pathology, 26, pages 33

El-Mofti et al.: American Journal of Pathology, vol. 79, no. 3, pp 579

## Description

This invention relates to novel medicaments for the treatment and prevention of liver damage caused or induced by drugs, toxic chemicals, poisons, radiation, alcohol and viruses. The known compound nicardipine of the formula

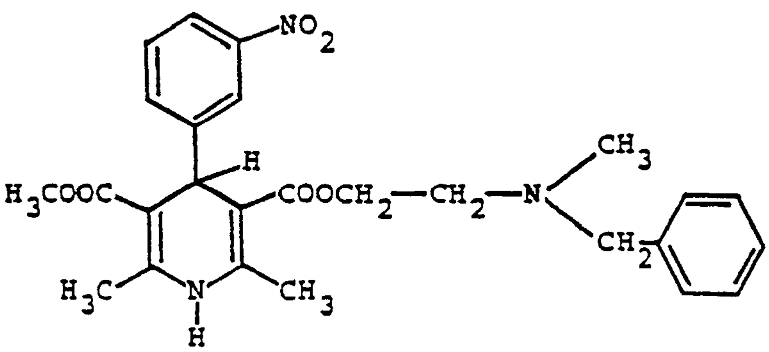

and nicardipine salts with non-toxic acids, particularly nicardipine hydrochloride, were found to be active in the prevention and treatment of liver damage and would, therefore, be useful for the manufacture of medicaments for treatment of viral hepatitis, alcoholic liver disease, drug, poison, radiation and chemothrapy induced liver disease and toxic hepatitis.

The liver is the largest and metabolically the most complex and active organ of the body. The functions of the liver are manyfold and include metabolism, synthesis, detoxification, secretion and storage. All nutrients, toxins, poisons, drugs and viruses pass through the liver and there they are metabolized, detoxified, or converted to other substances by the liver cells (hepatocytes). Therefore, any damage or injury to the hepatocytes causes disturbance of liver function with severe consequences for normal functioning of the whole body.

It is known from FR—A—2 248 028 that 2,6-dimethyl-3,5-dicarbonyl-1,4-dihydropyridine derivatives are useful as antioxidants for phospholipids in liver cells. This antioxidant activity of 1,4-dihydropyridine derivatives is confirmed in Chemical Abstracts 99, 207 523 n (1983) and Chemical Abstract 76, 107 917 a (1972). Further, in Chemical Abstracts 90, 115 049 y (1979) it is reported that nicardipine has a cyclic AMP phosphodiesterase inhibiting activity.

For clinical purposes, the liver can be considered in terms of blood supply, parenchymal cells, Kupffer cells and biliary passages. Specific liver diseases tend to affect these four components in a predictable manner, often with characteristic clinical, biochemical and histological consequences.

Symptons and signs of hepatic dysfunction are numerous and while some may be seen only in chronic liver disease, the others will be observed in both acute and chronic liver disorders.

The liver damage caused by viruses, drugs, toxic chemicals, poisons, alcohol or radiation are characterized by the elevation of the liver enzymes such as glutamic-pyruvic transaminase (GPT), glutamic-oxalic transaminase (GOT), alkaline phosphatase (AP), sorbital dehydrogenase (SDH) and in the level of bilirubin. These tests are most useful in determination of the character and the extent of liver damage and are generally part of routine evaluation of the patient with liver disease. For example, increased activity of serum alkaline phosphatase suggests mechanical obstruction of the extrahepatic biliary tree, an infiltrative lesion of the liver, early manifestation of cirrhosis or drug induced cholestasis. Increase in the serum transaminases (GPT and GOT) and sorbitol dehydrogenase activity is common in all kinds of liver disease and indicates hepatocyte injury. *The Merck Manual,* 13th Ed., Section 8:836 (1977). Therefore, any decrease in the elevated levels of these enzymes following the drug treatment would indicate that the drug is effective in treatment of the liver damage.

Jaundice, one of the most known and visible symptons of liver disorder, is caused by excess of circulating bilirubin. Any abnormality due to the liver disorder in the bilirubin metabolism will cause hyperbilirubinemia. Thus, any increase in the level of bilirubin is considered a good indicator of liver damage and the determination of serum bilirubin is a necessary part of the routine evaluation of a patient with liver disease.

*Ibid.*

The liver injury is also accompanied by the liver cell necrosis affecting particularly parenchymal liver cells. Such necrosis can be dertermined histologically and is an excellent indicator of the extent of liver injury. Moreover, the changes in the magnitude of the liver cells necrosis indicate whether the drug treatment was effective and to which extent.

Successful drug treatment of liver damage is, therefore, accompanied by:

1. reducing the elevated liver enzymes;
2. decreasing levels of bilirubin; and

3. diminishing the extent of liver cells necrosis.

Because of its importance, liver damage has been widely studied and models of simulating hepatic injuries similar or identical to the viral or chemical hepatitis were developed. The models of experimental hepatitis are numerous. Those, however, most resembling the actual viral or chemical hepatitis are those induced by hepatotoxins such as D-galactosamine, carbon tetrachloride or bromobenzene.

The administration of D-galactosamine to rats results in a reproducible liver cell injury. Moreover, the biochemical and morphological pattern of resulting liver cell injury is qualitatively related to the dose of D-galactosamine. Since the high dose of D-galactosamine induces a reversible structural and functional change in rat livers, it makes it anaylzable model for the study of effect of drugs and pharmaceuticals on the liver damage. Carbon tetrachloride is a known model hepatotoxin widely used for studies of acute chemical liver injuries. In the liver, carbon tetrachloride is converted by the liver cells to a metabolite which peroxidizes and alkylates tissue macromolecules. This conversion initiates the stereotypical events of acute lethal liver cell injury. Aside from D-galactosamine, the carbon tetrachloride model is considered most suitable for studies of liver injuries particularly those due to chronic alcholic ingestion.

The galactosamine and carbon tetrachloride hepatitis models are described, for example, in *Amer. J. Path.,* 79:579, (1975); *Virchows Arch. B. Cell. Path.,* 26:331 (1978); and in *Seminars in Liver Disease,* 1:143 (1981).

It is already known, for instance, by FR—A—2,248,028 or EP—B—0,087,156, that certain 1,4-dihydropyridine compounds possess a useful activity against liver disorders.

Using the above models, Applicant has found that nicardipine and nicardipine salts, particularly nicardipine hydrochloride, administered before the liver damage occurred, effectively prevent the increase in liver enzymes and bilirubin and is thus useful in obviating the liver damage. The nicardipine and nicardipine hydrochloride are equally effective in decreasing elevated liver enzymes and bilirubin when administered after the damage to the liver has already occurred and are, therefore, also useful for treatment of liver damage. These conclusions are supported by both biochemical and histological findings that the administration of nicardipine and particularly nicardipine hydrochloride, regardless whether before or after the liver injury occurs, was able to decrease the levels of GPT, GOT, SDH and bilirubin and also diminish the magnitude of the hepatocellular necrosis.

Compounds which are the subject of this invention, namely nicardipine and nicardipine salts are known peripheral vascular dilaters, anti-anginal agents and antihypertensives. Their preparation and above utility have been described in the U.S. Patent 3,985,758.

Hepato-protectivce properties of compounds somehow structurally related was disclosed in European Patent 0 087 156—A. Therefrom, however, it was not possible to predict any new effect of the nicardipine and its salts.

It has now been found that nicardipine and nicardipine salts, particularly nicardipine hydrochloride, are active in diminishing the symptons appearing during the liver damage and injury. Thus, medicaments containing these compounds significantly decrease the elevated levels of liver enzymes, such as, for example glutamic-oxalacetic transaminase (GOT), glutamic-pyruvic transaminase (GPT) and sorbitol dehydrogenase (SDH). These medicaments also lower the bilirubin level and decrease the magnitude of hepatocellular necrosis.

## Definitions

"Nicardipine" as used herein means the compound named as:

2,6-dimethyl-4-(3′-nitrophenyl)-1,4-dihydropyridine-3,5-dicarboxylic acid 3-β(N-benzyl-N-methylamino) ethyl ester 5 methyl ester (*U.S. Patent 3,985,758); or*

1,4-dihydro-2,6-dimethyl-4-(3-nitrophenyl)-3,5-pyridine-dicarboxylic acid methyl 2-[methyl-(phenylmethyl)-amino]ethyl ester (*Merck Index*); or

3,5-pyridinedicarboxylic acid, 1,4-dihydro-2,6-dimethyl-4(3-nitrophenyl)-, methyl 2-[methyl-(phenylmethyl)-amino]ethyl ester (*USAN*); or

2-(Benzylmethylamino)ethyl methyl 1,4-dihydro-2,6-dimethyl-4-(m-nitrophenyl)-3,5-pyridine dicarboxylate (*USAN*).

"Nicardipine salt" means a pharmaceutically acceptable non-toxic salt of the nicardipine.

"Pharmaceutically acceptable non-toxic salts" refers to pharmaceutically acceptable hydrogen-anion addition salts which do not adversely affect the pharmaceutical properties of the parent compounds. With respect to the addition salts, suitable inorganic anions include, for example, chloride, bromide, iodide, sulfate, phosphate and nitrate. Suitable organic anions include, for example, acetate, benzoate, lactate, picrate, propionate, butyrate, valerate, tartarate, maleate, fumarate, citrate, succinate, tosylate, ascorbate, nicotinate, adipate and gluconate.

The term "Mammals" means a class of warm-blooded verterbrates characterized by mammary glands, including but not limited to humans, laboratory or domestic animals such as dogs, cats, mice, rats or rabbits, and livestock.

"Treatment" covers any treatment of the disease in a mammal, particularly human, and includes:

(i) preventing the disease from occuring in a subject which may be predisposed to the disease but has not yet been diagnosed as having it;

(ii) inhibiting the disease, i.e. arresting the development of said disease; or

(iii) relieving the disease, ie. causing regression of the disease.

"Liver damage", "liver disorders", or "liver disease" as used herein mean hepatic and biliary disorders which are caused by viruses, drugs, toxic chemicals, poisons, radiation or alcohol and are meant to include all hepatic disease and disorders as described in *The Merch Manual,* 13th Ed., Section 8, pp:836—886 (1977).

The broadest aspect of this invention is given in the Summary of this Invention.

Most preferred compound useful in the method of the current invention is nicardipine hydrochloride.

Preparation procedures

Nicardipine and nicardipine salts are disclosed and their detailed preparation is described in U.S. Patent 3,985,758.

Sustained release pharmaceutical composition containing nicardipine salts is described in U.S. Patent 4,393,789.

The nicardipine in free base form may be converted to the acid addition salts by treating it with a stoichiometric excess of the appropriate organic or inorganic acid, such as, for example, phosphoric, pyruvic, hydrochloric or sulfuric acid and the like. Typically, the free base is dissolved in a polar organic solvent such as ethanol or methanol, and the acid added thereto. The temperature is maintained between about 0°C and 100°C. The resulting acid addition salt precipitates spontaneously or may be brought out of solution with a less polar solvent.

The acid addition salts of nicardipine may be decomposed to the corresponding free base by treating it with a stoichiometric excess of a suitable base, such as potassium carbonate or sodium hydroxide, typically in the presence of aqueous solvent, and at a temperature of between about 0°C and 100°C. The free base form is isolated by conventional means, such as extraction with an organic solvent.

Nicardipine salts may be interchanged by taking advantange of differential solubilities of the salts, volatilities or acidities of the acids, or by treating with the appropriately loaded ion exchange resin. For example, the interchange is effected by the reaction of a nicardipine salt with a slight stoichiometric excess of an acid of a lower pKa than the acid component of the starting salt. This conversion is carried out at a temperature between about 0°C and the boiling point of the solvent being used as the medium for the procedure.

Utility

This invention relates to a novel medicament for a treatment and prevention of liver damage caused or induced by drugs, toxic chemicals, poisons, radiation, alcohol and viruses. Nicardipine and nicardipine salts, particularly nicardipine hydrochloride, compounds previously known as peripheral and cerebral vascular dilators, antihypertensives and anti-anginal agents were now found to be active in prevention and treatment of liver disorders and would be, therefore, useful for treatment of such liver disorders as, for example:

1. viral hepatitis type A or type B, herpes, mononucleosis, rubella, mumps and coxsackie;
2. alcoholic liver disease, such as fatty liver, steatosis, hepatomegaly, intrahepatic obstructive jaundice, jaundice, portal hypertension, cirrhosis, fibrosis and acute hepatocellular failure;
3. drug induced liver disease, such as drug induced acute hepatitis, drug induced jaundice or cholestasis;
4. toxic hepatitis caused by such toxic agents as carbon tetrachloride and related hydrocarbons, industrial solvents and phosphorus or other toxic chemicals; or
5. poison hepatitis caused by mushroom poisoning, by poisoning with fungal products known as alfatoxins or by other poisons;
6. radiation and chemotherapy induced liver injury.

Administration

Administration of nicardipine or its salt, preferably nicardipine hydrochloride, can be via any of the accepted modes of administration suitable for treatment of the liver disorder. These methods include oral routes, parenteral routes such as intravenous, subcutaneous, intradermal, or intramuscular and other systemic routes of administration such as, for example, by suppositories.

The amount of nicardipine or its salt administered will, of course, be dependent on the subject being treated, on the severity of the affliction, on the manner of administration and on the judgement of the prescribing physician. However, an effective dosage is in the range of 0.001—5 mg/kg/day, preferably 0.03—1 mg/kg/day. For an average 70 kg human, this would amount to 0.07—350 mg/day, preferably 2—70 mg/day.

For oral administration, which is preferred, a pharmaceutical composition takes the form of solutions, suspensions, tablets, pills, capsules, powders and sustained release formulations.

Parenteral route of administration is the administration of drugs to a patient by injection under or through one or more layers of the skin or mucous membrane. Parenteral administration would preferably be reserved for crisis situations, wherein the subject is unable to swallow or administer the medication to himself.

Systemic administration via suppository is the administration of the drug in a solid but readily meltable cone or cylinder made of suitable pharmaceutical excipient. Suppository must be suitable for insertion into

a bodily passage or cavity, and is usually inserted into the rectum. This way of administration would be preferred in the patient with severe ingestion disturbance such as repeated vomiting usually occuring in cases of poisoning.

### Pharmaceutical Composition

Depending on the intended mode of administration, the manufacture of the pharmaceutical compositions in the form of solid, semi-solid or liquid dosage forms, such as, for example, tablets, pills, capsules, powders, liquids or suspensions, preferably in unit dosage forms suitable for single administration of precise doages may be performed in the usual way. The pharmaceutical compositions will include a conventional pharmaceutical carrier or excipient, nicardipine or the pharmaceutically acceptable salt, preferably nicardipine hydrochloride, as an active ingredient thereof. In addition, it may include other medicinal or pharmaceutical agents, carriers or adjuvants.

A pharmaceutical composition may contain 0.1%—95% of nicardipine or nicardipine salt, preferably 1%—70%. In any event, the composition or formulation to be administered will contain a quantity of nicardipine or nicardipine salt in an amount effective to alleviate the symptons of the subject being treated.

For solid pharmaceutical compositions, conventional non-toxic solid carriers include, for example, pharmaceutical grades of mannitol, lactose, starch, magnesium stearate, sodium saccharin, talcum, cellulose, glucose, sucrose and magnesium carbonate.

Liquid pharmaceutically administerable compositions can be prepared by dissolving or dispersing, or otherwise preparing a nicardipine or nicardipine salt, and mixing it optionally with a pharmaceutical adjuvant in a carrier, such as, for example, water, saline, aqueous dextrose, glycerol, ethanol, and the like, to thereby form a solution or suspension.

For parenteral administration, such as, for example, intravenous injections, the nicardipine or nicardipine salt is dissolved in a vehicle. Vehicles may be, for example, an aqueous vehicle such as sodium chloride injection, Ringer's injection, dextrose injection and others, a water miscible vehicle such as ethyl alcohol, polyethylene glycol of the liquid series or propylene glycol, or nonaqeuous vehicles such as corn oil, peanut oil or sesame oil. Vehicle will be buffered to the proper pH in order to stabilize a solution against chemical degradation and formed in such a way as to control isotonicity of injection. Other substances may also be added as antimicrobial or antioxidant agents.

A more recently devised approach for parenteral administration employs the implantation of a slow-release or sustained-release system, such that a constant level of dosage is maintained. See, e.g., U.S. Patent No. 3,710,795.

For systemic administration via suppository, the nicardipine or nicardipine salt may be formulated as suppositories using as the carrier traditional binders and carriers such as; for example, polyalkylene glycols, or triglycerides. Such suppositories may be formed from mixtures containing nicardipine or nicardipine salt in the range of 0.5%—10%; preferably 1—2%.

Methods of preparing various pharmaceutical compositions with a certain amount of active ingredient are known, or will be apparent, to those skilled in this art. For examples, see *Remington's Pharmaceutical Sciences,* Mack Publishing Company, Easton, Pennsylvania, 15th Edition (1975).

The following 2 pharmaceutical tests illustrate the surprising effect of medicaments according to the invention.

### Test 1

### Prevention of Experimental Liver Damage in Rats

This test illustrates the ability of a medicament of the current invention to prevent an experimentally induced liver damage. The procedure used in this test is the modified procedure of Lier et al, *Virchows Arch B. Cell Path.,* 26:331, (1978).

### Experimental protocol

The experimental liver damage was induced either with D-galactosamine or with carbon tetrachloride as the challenging agents. Liver damage caused by D-galactosamine resembled that seen after acute hepatitis. Carbon tetrachloride caused the liver damage resembled that of alcohol-induced liver injury.

Male Bantin-Kingman Sprague Dawley rats weighing between 220—220 grams were divided in six groups with 10 rats per group. Rats were starved 18 hours prior to sacrifice otherwise food and water were available *ad libitum.* The challenge was administered either only once at 24 hours prior sacrifice, or twice 24 hours and 18 hours prior sacrifice, depending on the regimen. The tested compound was administered in various defined intervals prior to sacrifice depending on the challenging agent and on the chosen regimen. At the time of the sacrifice the rats were anesthetized with ether, bled via the orbital sinus of the eye and then sacrificed. The collected blood was spun down and the plasma was saved for liver enzyme and/or liver metabolic function determination.

The levels of GPT, GOT, SDH and bilirubin were determined in controls and in experimental groups. The bilirubin and all enzymes were determined using the Autoanalyser Gemeni (manufactured by Electronucleonics, Inc). The instrument utilizes the modified methods of Walters and Gerarde, *Michrochem. J.,* 15:231 (1970) for determination of bilirubin and modified method of Henry, *Amer. J. Clin. Path.,* 34:381 (1960) for determination of GPT, GOT and SDH. Independently, these enzymes were also measured with Sigma colorimetric methods, *Sigma Diagnostic Kits and Reagents, Sigma Catalog* (1983).

Protocol — D-Galactosamine Challenge

*Challenge:* D-galactosamine:
Dose: 375 mg/kg, given twice;
Administered: 24 hours and 18 hours prior sacrifice;
Route of administration: intraperitoneal;
*Tested Compound:* Nicardipine Hydrochloride:
Dose: Four times 50 mg/kg/ml;
Administered: 36 hours, 25.5 hours, 19.5 hours and 12 hours prior to sacrifice;
Route of administration: peroral;
*Control vehicle:* Distilled water;
Dose: ml/kg

Results (Mean ± S.E.)

| Test | Controls | Nicardipine HCl | Significance |
|---|---|---|---|
| GPT | 828 ± 309 u/l | 86 ± 16 u/l | p<0.05 |
| GOT | 556 ± 170 u/l | 199 ± 28 u/l | p<0.07 NS |
| SDH | 200 ± 68 u/l | 41 ± 11 u/l | p<0.05 |
| Bilirubin | 0.39 ± 0.05 mg/dl | 0.21 ± 0.03 mg/dl | p<0.01 |

All values are expressed in units of enzyme per liter of plasma or in mg of bilirubin per deciliter of plasma.

Following the administration of 50 mg/kg of nicardipine hydrochloride 12 hours, 1.5 before, 1.5 after the first challenge and 6 hours after the second challenge with D-galactosamine, GPT, SDH and bilirubin were significantly lowered in plasma. GOT was also lower but, at least in this study, the decrease was not significant. In the subsequent study with the same experimental set up except with decreased dose of nicardipine hydrochloride to four times 25 mg/kg/ml, the decrease seen in the level of GOT was significant. The degree of significance was $p<0.05$. The absolute values for control group of 10 animals were 919 ± 299 units of GOT per liter of plasma versus 251 ± 63 units of GOT per liter of plasma for experimental group fo 8 animals. Postmorten histological examination of the liver supported the above biochemical findings. The extent of hepatocellular necrosis in controls was significantly larger than the extent of necrosis seen in the experimental liver.

These results show that the administration of the nicardipine hydrochloride before the experimental liver D-galactosamine injury was afflicted was able to reduce significantly the extent of such liver injury. It can be, therefore, concluded that the liver injury resembling that of the acute hepatitis responds to the pretreatment with the nicardipine hydrochloride and that such pretreatment can prevent the extensive damage of the liver by the viruses of other hepatotoxins.

Protocol — Carbon Tetrachloride Challenge

*Challenge:* Carbon tetrachloride:
Dose: 1.8 g/kg peroral;
Administered: 24 hours prior sacrifice;
Route of administration: peroral;
*Tested Compound:* Nicardipine hydrochloride:
Dose: 25 mg/kg/ml;
Administered: 48 hours, 36 hours, 25.5 hours and 18 hours and 12 hours before sacrifice;
Route of administration: peroral;
*Control vehicle:* 1% gelatin suspension medium containing hydroxypropylmethylcellulose, dioctyl sodium sulphosuccinate and benzylalcohol;
Dose: 1 ml/kg
Route of administration: peroral.

Results (Mean ± S.E.)

| Test | Controls | Nicardipine HCl | Significance |
|---|---|---|---|
| GPT | 333 ± 20 u/l | 204 ± 32 u/l | p<0.005 |

The results are expressed in units of GPT per liter of plasma.

Following the administration of 25 mg/kg of the nicardipine hydrochloride 24 hours, 12 hours adn 1.5 hours before challenge, and 6 and 12 hours after the challenge with the carbon tetrachloride, GPT was found to be significantly lower in the experimental groups of treated animals than in untreated controls.

The results of this study show that the liver injury resembling that of alcohol induced liver damage responds to the pre-treatment with nicardipine hydrochloride.

Test 2

Treatment of Experimental Liver Damage in Rats

This test illustrates the ability of the medicament of the current invention to prevent and to treat an experimentally induced liver damage.

Essentially, the procedure and experimental protocol used in this example are the same as used in the test except that each group of rats was injected only once with 25 mg/kg of nicardipine hydrochloride at intervals of 3 hours before challenge (Group 1), 1.5 hour before challenge (Group 2), 1 hour after the challenge (Group 3), 2 hours before the challenge (Group 4) and 4 hours after the challenge (Group 5). The animals in control Group were injected with distilled water in the same intervals. All animals were sacrificed 24 hours after the challenge.

The challenging agent used to induce experimental liver damage was D-galactosamine.

Protocol — D-Galactosamine Challenge

*Challenge:* D-galactosamine:
  Dose: 50 mg/kg;
  Administered: 24 hours prior sacrifice;
  Route of administration: intraperitoneal;

*Tested Compound:* Nicardipine Hydrochloride
  Dose: 25 mg/kg/ml;
  Administered: 27 hours, 25.5 hours, 23 hours, 22 hours and 20 hours prior sacrifice;
  Route of administration: peroral;

*Control vehicle:* 1% gelatin suspension medium 1 ml/kg.

Results (Mean ± S.E.)

| Group | Time prior or post sacrifice/hours | | GPT/units/ 1/plasma | Significance |
|---|---|---|---|---|
| (CO) | | | 2001 ± 407 | |
| (1) | 3.0 | prior | 458 ± 161 | p<0.002 |
| (2) | 1.5 | prior | 423 ± 165 | p<0.001 |
| (3) | 1.0 | post | 1620 ± 405 | NS |
| (4) | 2.0 | post | 686 ± 225 | p<0.01 |
| (5) | 4.0 | post | 959 ± 253 | p<0.04 |

All results are expressed in units of the enzyme per liter of the plasma.

The administration of nicardipine hydrochloride to the experimental animals 3 hours, and 1.5 hours prior to the inducement of the experimental liver injury prevented the increase in GPT level in both Groups (1) and (2) and thus prevented the development of the extensive liver injury which was seen in the control group. The administration of nicardipine hydrochloride to the experimental animals 1 hour (Group 3), 2 hours (Group 4) and 4 hours (Groups 5) after the inducement of the experimental liver injury was able to decrease the level of the GPT from approx 2001 units/l of plasma to 1620 units of GPT per liter of plasma in Group (3), to 686 units per liter of plasma in Group (4) and to 959 units per liter of plasma in Group (5).

The above results show that nicardipine hydrochloride was able not only to prevent the development of the liver injury following the challenge but also to treat already incurred liver injury.

In the following Examples for the manufacture of medicaments according to the invention nicardipine hydrochloride is used as active ingredient.

Example 1

| Ingredients | Quantity in mg per tablet |
|---|---|
| Active ingredient | 25 |
| cornstarch | 20 |
| lactose, spray-dried | 153 |
| magnesium stearate | 2 |

The above ingredients are thoroughly mixed and pressed into single scored tablets.

Example 2

| Ingredients | Quantity in mg per tablet |
|---|---|
| Active ingredient | 100 |
| lactose, spray-dried | 148 |
| magnesium stearate | 2 |

The above ingredients are mixed and introduced into a hard-shell gelatin capsule.

Example 3

| Ingredients | Quantity in mg per tablet |
|---|---|
| Active ingredient | 200 |
| cornstarch | 50 |
| lactose | 145 |
| magnesium stearate | 5 |

The above ingredients are mixed intimately and pressed into single scored tablets.

Example 4

| Ingredients | Quantity in mg per tablet |
|---|---|
| Active ingredient | 108 |
| lactose | 15 |
| cornstarch | 25 |
| magnesium stearate | 2 |

The above ingredients are mixed and introduced into a hard-shell gelatin capsule.

Example 5

| Ingredients | Quantity in mg per tablet |
|---|---|
| Active, ingredient | 150 |
| lactose | 92 |

The above ingredients are mixed and introduced into a hard-shell gelatin capsule.

Example 6

An injectable preparation buffered to a pH of 7 is prepared having the following composition:

| Ingredients | |
|---|---|
| Active, ingredient | 0.2 g |
| $KH_2PO_4$ buffer (0.4 M solution) | 2 ml |
| KOH (1 N) | q.s. to pH 7 |
| water (distilled, sterile) | q.s. to 20 ml |

Example 7

An oral suspension is prepared having the following composition:

| Ingredients | |
|---|---|
| Active ingredient | 0.1 g |
| fumaric acid | 0.5 g |
| sodium chloride | 2.0 g |
| methyl paraben | 0.1 g |
| granulated sugar | 25.5 g |
| sorbitol (70% solution) | 12.85 g |
| Veegum K® (Vanderbilt Co.) | 1.0 g |
| flavoring | 0.035 ml |
| colorings | 0.5 mg |
| distilled water | q.s. to 100 ml |

**Claims**

1. The use of 2,6-dimethyl-4-(3'-nitrophenyl)-1,4-dihydropyridine-3,5-dicarboxylic acid 3-ß-(N-benzyl-N-methylamino)ethyl-ester-5-methyl ester — nicardipine — or a pharmaceutically acceptable non-toxic salt thereof for the manufacture of a medicament for the prevention and treatment of liver damage in mammals.

2. The use of 2,6-dimethyl-4-(3'-nitrophenyl)-1,4-dihydropyridine-3,5-dicarboxylic acid 3-ß-(N-benzyl-N-methylamino)ethyl-ester-5-methyl ester hydrochloride for the manufacture of a medicament for the prevention and treatment of liver damage in mammals.

**Patentansprüche**

1. Verwendung von 2,6-Dimethyl-4-(3'-nitrophenyl)-1,4-dihydropyrridin-3,5-dicarbonsäure-3-ß-(N-benzyl-N-methylamino)-ethylester-5-methylester-nicardipin oder eines pharmazeutisch annehmbaren nicht toxischen Salzes davon zur Herstellung eines Medikaments zur Vorbeugung und Behandlung von Leberschäden in Säugetieren.

2. Verwendung von 2,6-Dimethyl-4-(3'-nitrophenyl)-1,4-dihydropyrridin-3,5-dicarbonsäure-3-ß-(N-benzyl-N-methylamino)ethylester-5-methylester-hydrochlorid zur Herstellung eines Medikaments zur Vorbeugung und Behandlung von Leberschäden in Säugetieren.

**Revendications**

1. Application de 3-β-(N-benzyl-N-méthylamino)éthylester-5-méthyl-ester de l'acide 2,6-diméthyl-4-(3'-nitrophényl)-1,4-dihydropyridine-3,5-dicarboxylique — nicardipine — ou d'un de ses sels non toxiques pharmaceutiquement acceptables à la préparation d'un médicament pour la prévention et la traitement des dommages au foie chez les mammifères.

2. Application de chlorhydrate de 3-β-(N-benzyl-N-méthylamino)éthylester-5-méthyl-ester de l'acide 2,6-diméthyl-4-(3'-nitrophényl)-1,4-dihydropyridine-3,5-dicarboxylique à la préparation d'un médicament pour la prévention et le traitement des dommages au foie chez les mammifères.